# EUROPÄISCHE PATENTSCHRIFT

(11) **EP 2 162 420 B1**
(45) Veröffentlichungstag und Bekanntmachung des Hinweises auf die Patenterteilung: **07.09.2016**
(21) Anmeldenummer: 08759458.6
(22) Anmeldetag: 08.05.2008
(51) Int. Cl.: C07C 67/03, C07C 69/54

(54) **VERFAHREN ZUR HERSTELLUNG VON ALLYLMETHACRYLAT**
METHOD FOR SYNTHESIZING ALLYL METHACRYLATE
PROCEDE DE PRODUCTION DE METHACRYLATE D'ALLYLE

(30) Priorität: 05.07.2007 DE 102007031468
(43) Veröffentlichungstag der Anmeldung: 17.03.2010
(73) Patentinhaber: Evonik Röhm GmbH, 64293 Darmstadt (DE)
(72) Erfinder: SCHMITT, Bardo, 55252 Mainz (DE); PROTZMANN, Guido, 64625 Bensheim (DE); SCHÜTZ, Thorben, 64342 Seeheim-Jugenheim (DE); TRAUTHWEIN, Harald, 68642 Bürstadt (DE); MARTIN, Reinhold, 64732 Bad König (DE); KNEBEL, Joachim, 64665 Alsbach-Hähnlein (DE); SANDER, Ingo, 201108 Ming Hang District Shanghai (CN); GOTTMANN, Klaus, 64646 Heppenheim (DE); KEHR, Thomas, 64367 Muehltal (DE); BATHEN, Dieter, 47058 Duisburg (DE); MAUL, Christian, 67435 Neustadt a. d. W. (DE)
(86) Internationale Anmeldenummer: PCT/EP2008/055669
(87) Internationale Veröffentlichungsnummer: WO 2009/003746

(56) Entgegenhaltungen:
- EP-A- 1 078 913
- US-A- 4 202 990

## Beschreibung

Die vorliegende Erfindung betrifft Verfahren zur Herstellung von Allylmethacrylat.

Allylmethacrylat dient unter anderem als Intermediat zur Herstellung von Silylgruppen-haltigen Methacrylaten. Dementsprechend sind vielfältige Methoden bekannt, um diese Verbindung zu erhalten. Hierzu gehören insbesondere Verfahren, bei denen Allylalkohol mit Methacrylaten, beispielsweise Methylmethacrylat oder Ethylmethacrylat, umgesetzt wird. Zur Verbesserung der Ausbeute und der Selektivität der Reaktion können unterschiedliche Katalysatoren eingesetzt werden.

Beispielsweise können Säuren oder Basen eingesetzt werden, um die Umesterung zu katalysieren. Derartige Reaktionen werden beispielsweise in CN 1410412 oder DE 34 23 441 dargelegt. Bei Einsatz dieser Katalysatoren muss allerdings mit Nebenreaktionen gerechnet werden, wie beispielsweise der Michael-Addition, die sowohl die Reinheit des gewünschten Allylmethacrylats als auch die Ausbeute schmälert.

Gemäß der Druckschrift JP 11222461 kann die Umesterung von Methylmethacrylat mit Allylalkohol durch Titanalkoholate katalysiert werden. Hierbei werden insbesondere stickstoffhaltige Polymerisationsinhibitoren eingesetzt, die jedoch im Allylmethacrylat unerwünscht sind.

Weiterhin beschreibt die Druckschrift JP-01-258642 die Umsetzung von Methylmethacrylat mit Allylalkohol in Gegenwart von Titanalkoholaten. Hierbei werden sauerstoffhaltige Polymerisationsinhibitoren eingesetzt.

Darüber hinaus beschreibt die Druckschrift DE 28 05 702 die Herstellung von Estern ungesättigter Carbonsäuren. Zur Katalyse der beschriebenen Reaktionen können insbesondere Verbindungen eingesetzt werden, die Zirkonium und/oder Calcium enthalten. Zu den besonders geeigneten Katalysatoren gehört insbesondere Zirkoniumacetylacetonat. Die Herstellung von Allylmethacrylat wird jedoch nicht explizit beschrieben. Die Umsetzungen führen zu hohen Ausbeuten von ca. 98%, bezogen auf den eingesetzten Alkohol. Hieraus ergibt sich jedoch, dass das Produkt beachtliche Mengen an Nebenprodukten enthält.

Die Herstellung von Silylgruppen-haltigen Methacrylaten aus Allylalkohol erfordert eine sehr hohe Reinheit der Edukte, da Verunreinigungen, z.B. Allylalkohol und Wasser den für die Synthese des Silanmethacrylats verwendeten Pt-Katalysator deaktivieren können. In gleicher Weise stören stickstoffhaltige Nebenkomponenten. Allylmethacrylat, das für diese Zwecke kommerziell als Edukt vertrieben wird, darf daher höchstens 200 ppm Allylalkohol enthalten, wobei ein möglichst geringer Gehalt an Allylalkohol erstrebenswert ist. Um diese Anforderungen zu erfüllen, müssen die gemäß dem Stand der Technik erhaltenen Produkte aufwendig aufgereinigt werden.

In Anbetracht des Standes der Technik war es nun Aufgabe der vorliegenden Erfindung, ein Verfahren zur Herstellung von Allylmethacrylat zur Verfügung zu stellen, bei dem das Produkt mit einer sehr hohen Reinheit erhalten wird. Insbesondere sollte das erhaltene Allylmethacrylat nur sehr geringe Mengen an Allylalkohol und/oder Wasser enthalten.

Eine weitere Aufgabe der Erfindung bestand darin, ein Verfahren zu schaffen, bei dem Allylmethacrylat sehr selektiv erhalten werden kann.

Darüber hinaus war es Aufgabe der vorliegenden Erfindung, Verfahren zur Herstellung von Allylmethacrylat zur Verfügung zu stellen, die einfach und kostengünstig durchgeführt werden können. Hierbei sollte das Produkt möglichst in hohen Ausbeuten und, insgesamt gesehen, unter geringem Energieverbrauch erhalten werden. Darüber hinaus sollte die Umsetzung insbesondere ohne stickstoffhaltige Polymerisationsinhibitoren durchgeführt werden können.

Gelöst werden diese sowie weitere nicht explizit genannte Aufgaben, die jedoch aus den hierin einleitend diskutierten Zusammenhängen ohne Weiteres ableitbar oder erschließbar sind, durch Verfahren mit allen Merkmalen des Patentanspruchs 1. Zweckmäßige Abwandlungen der erfindungsgemäßen Verfahren werden in den auf Anspruch 1 rückbezogenen abhängigen Ansprüchen unter Schutz gestellt.

Gegenstand der vorliegenden Erfindung ist dementsprechend ein Verfahren zur Herstellung von Allylmethacrylat, umfassend die Umsetzung von Allylalkohol mit einem Ester der Methacrylsäure, wobei die Umsetzung durch Zirkoniumacetylacetonat katalysiert wird.

Hierdurch gelingt es auf nicht vorhersehbare Weise ein Verfahren zur Herstellung von Allylmethacrylat zur Verfügung zu stellen, bei dem das Produkt mit einer sehr hohen Reinheit erhalten wird. Überraschend enthält das erhaltene Produkt nur sehr geringe Mengen an Allylalkohol und/oder Wasser.

Des Weiteren ermöglicht das erfindungsgemäße Verfahren eine besonders selektive Herstellung von Allylmethacrylat.

Weiterhin kann das erfindungsgemäße Verfahren einfach und kostengünstig durchgeführt werden, wobei das Produkt in hohen Ausbeuten und, insgesamt gesehen, unter geringem Energieverbrauch erhalten werden kann. Darüber hinaus kann die Umsetzung insbesondere ohne stickstoffhaltige Polymerisationsinhibitoren durchgeführt werden.

Erfindungsgemäß wird Allylmethacrylat hergestellt. Allylmethacrylat (2-Methylpropensäurepropenylester) ist seit langem bekannt und besitzt die CAS-Nummer 96-05-9.

Zur Herstellung von Allylmethacrylat wird erfindungsgemäß Allylalkohol (2-Propen-1-ol) eingesetzt, der kommerziell z.B. von Fa. Lyondell erhältlich ist. Die CAS-Nummer von Allylalkohol ist 107-18-6.

Gemäß der vorliegenden Erfindung wird Allylalkohol mit einem Ester der Methacrylsäure umgesetzt. Besonders geeignete Methacrylate werden insbesondere von Alkoholen mit 1 bis 4 Kohlenstoffatomen gebildet. Zu diesen gehören insbesondere Methanol, Ethanol, n-Propanol, iso-Propanol, n-Butanol und tert.-Butanol. Besonders bevorzugt wird insbesondere Ethylmethacrylat oder Methylmethacrylat eingesetzt, wobei Methylmethacrylat ganz besonders bevorzugt ist.

Das Gewichtsverhältnis von Allylalkohol zum Ester der Methacrylsäure liegt vorzugsweise im Bereich von 1:1,5 bis 1:10, besonders bevorzugt 1:2,5 bis 1:5 und ganz besonders bevorzugt im Bereich von 1:3 bis 1:4. Ein zu kleiner Esterüberschuß kann die Reaktionsgeschwindigkeit vermindern, ein zu großer ist wirtschaftlich nicht sinnvoll da er das nutzbare Kesselvolumen schmälert.

Erfindungsgemäß wird zur Katalyse der vorliegenden Umesterung Zirkoniumacetylacetonat eingesetzt. Die CAS-Nummer von Zirkoniumacetylacetonat ist 17501-44-9. Die Herstellung von Zirkoniumacetylacetonat aus Acetylaceton (Pentan-2,4-dion) und Zirconiumverbindungen ist beispielsweise in Houben-Weyl, Methoden der organischen Chemie, 4. Auflage, Bd. VI/2, 1963, Seiten 53-55 und 58 bis 61 sowie in A.E. Martell, M. Calvin, "Die Chemie der Metallchelatverbindungen" (1958) beschrieben. Vorteilhaft können 0,2 bis 5 mmol, besonders bevorzugt 0,5 bis 2 mmol Zirkoniumacetylacetonat pro mol Allylalkohol eingesetzt werden. Die Herstellung des Katalysators kann auch in situ erfolgen, wobei die Ausgangsstoffe vor oder während der Umesterung der Reaktionsmischung beigefügt werden können.

Die Umsetzung kann bei Über- oder Unterdruck erfolgen. Gemäß einer besonders zweckmäßigen Abwandlung der vorliegenden Erfindung kann die Umesterung bei einem Druck im Bereich von 200 bis 2000 mbar, besonders bevorzugt im Bereich von 500 bis 1300 mbar. durchgeführt werden.

Die Reaktionstemperatur kann, insbesondere in Abhängigkeit des Druckes, ebenfalls in einem weiten Bereich liegen. Gemäß einer bevorzugten Ausführungsform der vorliegenden Erfindung erfolgt die Umsetzung vorzugsweise bei einer Temperatur im Bereich von 80°C bis 120°C, besonders bevorzugt 95 bis 115 °C.

Überraschend können besondere Vorteile erzielt werden, falls die Temperatur, bei der die Umsetzung erfolgt, im Verlauf der Umsetzung erhöht wird. Gemäß dieser bevorzugten Abwandlung des erfindungsgemäßen Verfahrens kann die Temperatur zu Beginn der Umsetzung, insbesondere bis zu einem Umsatz von 80%, bevorzugt bis zu einem Umsatz von 70%, bezogen auf das Gewicht des eingesetzten Allylalkohols, vorzugsweise im Bereich von 90°C bis 100°C und gegen Ende der Umsetzung, insbesondere nach einem Umsatz von 80%, bevorzugt nach einem Umsatz von 90%, bezogen auf das Gewicht des eingesetzten Allylalkohols, im Bereich von 105 °C bis 115 °C liegen.

Die Umesterung kann sowohl kontinuierlich als auch chargenweise durchgeführt werden. Es ist auch möglich, einen Teil des zur Umesterung eingesetzten Methacrylsäureesters nicht bereits vor Beginn der Reaktion vorzulegen, sondern erst während der Umsetzung zuzudosieren. Das erfindungsgemäße Verfahren kann in Substanz, d.h. ohne Verwendung eines weiteren Lösungsmittels durchgeführt werden. Falls gewünscht kann auch ein inertes Lösungsmittel eingesetzt werden. Hierzu gehören unter anderem Benzin, Benzol, Toluol, n-Hexan, Cyclohexan und Methylisobutylketon (MIBK), Methylethylketon (MEK).

Bei einer besonders zweckmäßigen Varianten der erfindungsgemäßen Umesterung werden sämtliche Komponenten, wie beispielsweise der Allylalkohol, der Methacrylsäureester sowie der Katalysator, gemischt, wonach diese Reaktionsmischung bis zum Sieden erwärmt wird. Hierbei wird zunächst Wasser, das in dem Alkohol enthalten sein kann, azeotrop mit dem Ester der Methacrylsäure abgetrennt. Anschließend kann der frei werdende Alkohol destillativ, beispielsweise Methanol oder Ethanol, gegebenenfalls azeotrop mit Methylmethacrylat oder Ethylmethacrylat, aus der Reaktionsmischung entfernt werden.

Gemäß einer besonderen Abwandlung der vorliegenden Umsetzung beträgt der Wasseranteil im Allylmethacrylat vorzugsweise höchstens 0,1%, besonders bevorzugt höchstens 0,02%, bezogen auf das Gewicht der Zusammensetzung.

Die Reaktionszeiten sind unter anderem von den gewählten Parametern, wie zum Beispiel Druck und Temperatur, abhängig. Sie liegen aber im Allgemeinen im Bereich von 1 bis 24 Stunden, bevorzugt von 3 bis 12 Stunden und ganz besonders bevorzugt 6 bis 9 Stunden. Bei kontinuierlichen Verfahren liegen die Verweilzeiten im Allgemeinen im Bereich von 0,5 bis 24 Stunden, bevorzugt von 1 bis 12 Stunden und ganz besonders bevorzugt 2 bis 3 Stunden. Weitere Hinweise in Bezug auf die Reaktionszeiten kann der Fachmann den beigefügten Beispielen entnehmen.

Vorzugsweise kann die Reaktion unter Rühren stattfinden, wobei die Rührgeschwindigkeit besonders bevorzugt im Bereich von 50 bis 2000 Upm, ganz besonders bevorzugt im Bereich von 100 bis 500 Upm liegen kann.

Der pH-Wert kann in einem weiten Bereich liegen. Zweckmäßig kann die Umsetzung bei einem pH-Wert im Bereich von 5 bis 9, vorzugsweise 6 bis 8 durchgeführt werden.

Um eine unerwünschte Polymerisation der Methacrylate zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tert-butylbrenzcatechin, Phenothiazin, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Besonders zweckmäßig sind unter anderem Polymerisationsinhibitoren, die keinen Stickstoff enthalten. Vorzugsweise werden insbesondere Phenole als Polymerisationsinhibitor eingesetzt. Besonders überraschende Vorteile können bei Verwendung von Mischungen erzielt werden, die Hydrochinon und Hydrochinonmonomethylether umfassen. Bezogen auf das Gewicht der gesamten Reaktionsmischung kann der Anteil der Inhibitoren einzeln oder als Mischung im Allgemeinen 0,01 - 0,5 % (wt/wt) betragen. Dabei ist es zweckmäßig, nicht nur den Reaktionsbehälter, sondern auch die Kolonne und gegebenenfalls die Kondensatorflächen mit Inhibitoren zu beschicken, die man beispielsweise in den Kolonnenrücklauf dosieren kann.

Zur Inhibierung kann des Weiteren Sauerstoff verwendet werden. Hierbei kann dieser zum Beispiel in Form von Luft eingesetzt werden, wobei die Mengen vorteilhaft so dosiert werden, dass der Gehalt in der Gasphase über dem Reaktionsgemisch unterhalb der Explosionsgrenze bleibt. Besonders bevorzugt sind hierbei Luftmengen im Bereich von 0,05 bis 0,5 l pro Stunde und Mol Allylalkohol. Bei Chargenverfahren kann sich diese Menge auf die ursprünglich eingesetzte Menge an Allylalkohol beziehen. Bei kontinuierlichen Verfahren kann sich diese Menge auf die zugeführte Menge an Allylalkohol beziehen. Ebenso können Inertgas-Sauerstoff-Gemische, z.B. Stickstoff-Sauerstoff-, Argon-Sauerstoff bzw. Kohlendioxid-Sauerstoff-Gemische eingesetzt werden.

Gemäß einer besonderen Ausgestaltung der vorliegenden Erfindung kann zur Inhibierung eine Kombination von Sauerstoff mit mindestens einem Phenol, vorzugsweise Hydrochinon und/oder Hydrochinonmonomethylether, eingesetzt werden.

Entsprechend einer zweckmäßigen Ausführungsform der vorliegenden Erfindung kann der aus dem eingesetzten Methacrylat freigesetzte Alkohol, beispielsweise Methanol und/oder Ethanol, durch Destillation abgetrennt werden. Hierbei kann vorteilhaft beispielsweise eine Mischung abgetrennt werden, die Methylmethacrylat und Methanol enthält. Überraschend kann mit Vorteil ein Teil der abgetrennten Mischung in den folgenden Ansatz zurückgeführt werden. Gemäß dieser Abwandlung kann der zurückführbare Anteil der abgetrennten Mischung zu Ende der Reaktion, insbesondere nach einem Umsatz von 80%, bevorzugt nach einem Umsatz von 90% des eingesetzten Allylalkohols, erhalten werden. Beispielsweise kann der Anteil der zurückgeführten Mischung zu Beginn des folgenden Ansatzes im Bereich von 40 bis 60%, bezogen auf dieGesamteinwaage an umzuesterndem Methacrylsäureester liegen.

Bei Chargenverfahren kann gegen Ende der Reaktion überschüssiges Edukt, insbesondere der nicht umgesetzte Ester der Methacrylsäure durch Destillation abgetrennt werden. Auch dieser kann im nächsten Batch ohne weitere Reinigung wieder eingesetzt werden.
Das zu Beginn der Umsetzung erhaltene methanol- oder ethanolreiche Destillat kann ebenfalls recycliert werden, beispielsweise durch Einarbeitung in eine im Verbund betriebene Anlage zur Herstellung des umzuesternden Methacrylatesters.

Eine geeignete Anlage zur Durchführung der vorliegenden Umesterung kann beispielsweise einen Rührkesselreaktor mit Rührwerk, Dampfheizung, Destillationskolonne und Kondensator umfassen. Derartige Anlagen sind an sich bekannt und beispielsweise in Ullmanns Encyclopedia of Industrial Chemistry (6. Auflage), Verlag Wiley-VCH ,Weinheim 2003, Band 10, Seite 647, beschrieben. Die Größe der Anlage ist von der herzustellenden Menge an Allylmethacrylat abhängig, wobei das vorliegende Verfahren sowohl im Labormaßstab als auch in großtechnischem Maßstab durchgeführt werden kann. Gemäß einem besonderen Aspekt kann der Rührkesselreaktor dementsprechend ein Kesselvolumen im Bereich von 1m³ bis 20 m³, bevorzugt 3 m³ bis 10 m³ aufweisen. Das Rührwerk des Reaktorkessels kann insbesondere in Form eines Ankerrührers, Impellers, Schaufel- oder Inter-MIG-rührers ausgestaltet sein.

Die Aufgabe der Destillationskolonne ist es, sicherzustellen, daß ein methanol- bzw. ethanolreiches Azeotrop abgeführt wird, um die Verluste an zwangsläufig mit ausgetragenem Eduktester zu minimieren.

Die Destillationskolonne kann ein, zwei oder mehr Trennstufen besitzen. Als Anzahl der Trennstufen wird die Anzahl der Böden bei einer Bodenkolonne oder die Anzahl der theoretischen Trennstufen im Fall einer Packungskolonne oder eine Kolonne mit Füllkörpern bezeichnet. Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz). Durch die umsatzabhängige Anpassung des Rücklaufverhältnis gelingt es beispielsweise, bei Verwendung von Methylmethacrylat über weite Bereiche des Umsatzes einen Methanolanteil im Destillat einzustellen, der oberhalb von 60% liegt.

Zu den geeignete Kondensatoren, die in der Anlage zur Durchführung der vorliegenden Umesterung enthalten sein kann, gehören unter anderem Platten- und Rohrbündelwärmetauscher.

Nach Beendigung der Umsetzung genügt das erhaltene Allylmethacrylat bereits vielfach den zuvor dargelegten hohen Anforderungen, so dass eine weitere Aufreinigung vielfach nicht notwendig ist. Zur weiteren Qualitätssteigerung und insbesondere der Katalysatorabtrennung kann die erhaltene Mischung durch bekannte Verfahren aufgereinigt werden. Wegen der Polymerisationsneigung des Monomeren sind Destillationsverfahren angeraten, bei denen die thermische Belastung des zu destillierenden Stoffes minimiert ist. Gut geeignet sind Vorrichtungen, bei denen das Monomer aus einer dünnen Schicht heraus kontinuierlich verdampft wird, wie Fallfilmverdampfer und Verdampfer mit einem rotierenden Wischersystem. Auch Kurzwegverdampfer können eingesetzt werden. Solche Vorrichtungen sind bekannt (Ullmanns Encyclopedia of Industrial Chemistry (6. Auflage), Verlag Wiley-VCH ,Weinheim 2003, Band 36, Seite 505). So kann beispielsweise eine Destillation durchgeführt werden, bei der ein kontinuierlicher Verdampfer mit rotierendem Wischersystem und aufgesetzter Kolonne eingesetzt werden kann. Diese Destillation kann beispielsweise bei Druck im Bereich von 40 bis 60 mbar und einer Verdampfertemperatur von 110°C bis 130°C durchgeführt werden.

Überraschend gelingt es durch die erfindungsgemäßen Maßnahmen ein Verfahren bereitzustellen, bei dem Allylmethacrylat erhalten werden kann, das vorzugsweise weniger als 0,04 %, besonders bevorzugt weniger als 0,02 % und ganz besonders bevorzugt weniger als 0,01 % Allylalkohol enthält, bezogen auf das Gewicht der Zusammensetzung.

Nachfolgend soll die vorliegende Erfindung anhand von Beispielen und Vergleichsbeispielen erläutert werden, ohne dass hierdurch eine Beschränkung erfolgen soll.

### Vergleichsbeispiel 1

In einem 7m³ - Rührkesselreaktor mit Rührwerk, Dampfheizung, Destillationskolonne und Kondensator werden 850 kg Allylalkohol, 4800 kg Methacrylsäuremethylester (MMA), 0,68 kg Phenothiazin sowie 0,22 kg N-N'-Diphenyl-p-phenylendiamin als Inhibitoren und als Katalysator 34 kg Lauryltitanat vereint und unter Einleiten von Luft gerührt. Man erhitzt auf 95°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 1:1 das Methanol-MMA-Gemisch ab. Innerhalb von 8 h wird das Rücklaufverhältnis bis auf 4,5:1 der abnehmenden Methanolentwicklung angepaßt. Bei einer Sumpftemperatur von 115 °C ist die Reaktion beendet und man zieht überschüssiges MMA im Vakuum ab, wobei man den Druck allmählich bis auf 5 Torr reduziert. Wenn kein MMA mehr abdestilliert, wird das Vakuum aufgehoben. Der Kesselinhalt besteht aus 1780 kg Allylmethacrylat, das noch 4000 ppm Allylalkohol und 1700 ppm Methylmethacrylat enthält (gaschromatographisch ermittelt).

### Vergleichsbeispiel 2

In einem 7m³ - Rührkesselreaktor mit Rührwerk, Dampfheizung, Destillationskolonne und Kondensator werden 1160 kg Allylalkohol, 4800 kg Methacrylsäuremethylester (MMA), 1,79 kg Hydrochinon sowie 0,34 kg Hydrochinonmonomethylether als Inhibitoren und als Katalysator 34 kg Lauryltitanat vereint und unter Einleiten von Luft gerührt. Man erhitzt auf 95°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 1:1 das Methanol-MMA-Gemisch ab. Innerhalb von 8 h wird das Rücklaufverhältnis bis auf 4,5:1 der abnehmenden Methanolentwicklung angepaßt. Bei einer Sumpftemperatur von 115 °C ist die Reaktion beendet und man zieht überschüssiges MMA im Vakuum ab, wobei man den Druck allmählich bis auf 5 Torr reduziert. Wenn kein MMA mehr abdestilliert, wird das Vakuum aufgehoben. Der Kesselinhalt besteht aus 2500 kg Allylmethacrylat, das noch 1200 ppm Allylalkohol und 7400 ppm Methylmethacrylat enthält (gaschromatographisch ermittelt).

### Beispiel 1

In einem 7m³ - Rührkesselreaktor mit Rührwerk, Dampfheizung, Destillationskolonne und Kondensator werden 1275 kg Allylalkohol, 4800 kg Methacrylsäuremethylester (MMA), 1,8 kg Hydrochinon sowie 0,34 kg Hydrochinonmonomethylether als Inhibitoren und als Katalysator 7,7 kg Zirkoniumacetylacetonat vereint und unter Einleiten von Luft gerührt. Man erhitzt auf 95°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 1:1 das Methanol-MMA-Gemisch ab. Innerhalb von 8 h wird das Rücklaufverhältnis bis auf 4,5:1 der abnehmenden Methanolentwicklung angepaßt. Bei einer Sumpftemperatur von 115 °C ist die Reaktion beendet und man zieht überschüssiges MMA im Vakuum ab, wobei man den Druck allmählich bis auf 5 Torr reduziert. Wenn kein MMA mehr abdestilliert, wird das Vakuum aufgehoben. Der Kesselinhalt besteht aus 2500 kg Allylmethacrylat, das nur noch 30 ppm Allylalkohol und 4280 ppm Methylmethacrylat enthält (gaschromatographisch ermittelt).

### Beispiel 2

In einem 7m³ - Rührkesselreaktor mit Rührwerk, Dampfheizung, Destillationskolonne und Kondensator werden 1326 kg Allylalkohol, 4800 kg Methacrylsäuremethylester (MMA), 1,8 kg Hydrochinon sowie 0,34 kg Hydrochinonmonomethylether als Inhibitoren und als Katalysator 7,7 kg Zirkoniumacetylacetonat vereint und unter Einleiten von Luft gerührt. Man erhitzt auf 95°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 1:1 das Methanol-MMA-Gemisch ab. Innerhalb von 8 h wird das Rücklaufverhältnis bis auf 4,5:1 der abnehmenden Methanolentwicklung angepaßt. Bei einer Sumpftemperatur von 115 °C ist die Reaktion beendet und man zieht überschüssiges MMA im Vakuum ab, wobei man den Druck allmählich bis auf 5 Torr reduziert. Wenn kein MMA mehr abdestilliert, wird das Vakuum aufgehoben. Der Kesselinhalt besteht aus 2740 kg Allylmethacrylat, das nur noch 10 ppm Allylalkohol und 4120 ppm Methylmethacrylat enthält (gaschromatographisch ermittelt).

### Beispiel 3

In einem 7m³ - Rührkesselreaktor mit Rührwerk, Dampfheizung, Destillationskolonne und Kondensator werden 1340 kg Allylalkohol, 4880 kg Methacrylsäuremethylester (MMA), 1,8 kg Hydrochinon sowie 0,34 kg Hydrochinonmonomethylether als Inhibitoren und als Katalysator 7,7 kg Zirkoniumacetylacetonat vereint und unter Einleiten von Luft gerührt. Man erhitzt auf 95°C Sumpftemperatur, wobei die Kolonne zunächst unter vollständigem Rücklauf betrieben wird. Sobald die Temperatur am Kolonnenkopf unter 70°C fällt, zieht man unter einem Rücklaufverhältnis von 1:1 das Methanol-MMA-Gemisch ab. Gleichzeitig dosiert man 700 kg MMA innerhalb von 4 h mit einer Rate in den Ansatz, die der pro Zeit ausgetragenen Methanol-MMA-Menge entspricht. Innerhalb von 8 h wird das Rücklaufverhältnis bis auf 4,5:1 der abnehmenden Methanolentwicklung angepaßt. Bei einer Sumpftemperatur von 117 °C ist die Reaktion beendet und man zieht überschüssiges MMA im Vakuum ab, wobei man den Druck allmählich bis auf 5 Torr reduziert. Wenn kein MMA mehr abdestilliert, wird das Vakuum aufgehoben. Der Kesselinhalt besteht aus 2680 kg Allylmethacrylat, das nur noch 50 ppm Allylalkohol und 1160 ppm Methylmethacrylat enthält (gaschromatographisch ermittelt).

### Destillative Reinigung des Allylmethacrylats:

In einen kontinuierlichen Verdampfer (Fläche 3,5 m²) mit rotierendem Wischersystem und aufgesetzter Kolonne werden bei 50 mbar Druck und 120°C Verdampfertemperatur 450 kg / h Rohallylmethacrylat eingespeist. Am Kopf der Kolonne stellt sich eine Temperatur von 60°C ein. Man zieht 425 kg / h Destillat ab, bestehend aus reinem Allylmethacrylat, das zur späteren Lagerung mit 50 ppm Hydrochinonmonomethylether stabilisiert wird.

### Zusammensetzung (gaschromatographisch ermittelt)

a) ausgehend von Rohstoff aus Vergleichsbeispiel 2:
   99,55 % Allylmethacrylat, 0,37 % MMA, 0,033 % Allylalkohol Wassergehalt (ermittelt durch Karl-Fischer-Titration): 350 ppm
b) ausgehend von Rohstoff aus erfinderischem Beispiel 1:
   99,71 % Allylmethacrylat, 0,22 % MMA, 0,006 % Allylalkohol Wassergehalt (ermittelt durch Karl-Fischer-Titration): 110 ppm
c) ausgehend von Rohstoff aus erfinderischem Beispiel 3:
   99,88 % Allylmethacrylat, 0,11 % MMA, 0,005 % Allylalkohol Wassergehalt (ermittelt durch Karl-Fischer-Titration): 50 ppm

## Patentansprüche

1. Verfahren zur Herstellung von Allylmethacrylat, umfassend die Umsetzung von Allylalkohol mit einem Ester der Methacrylsäure, **dadurch gekennzeichnet, dass** die Umsetzung durch Zirkoniumacetylacetonat katalysiert wird.

2. Verfahren gemäß Anspruch 1, **dadurch gekennzeichnet, dass** das Allylmethacrylat weniger als 0,02 % Allylalkohol enthält.

3. Verfahren gemäß mindestens einem der vorhergehenden Ansprüche, **dadurch gekennzeichnet, dass** das Gewichtsverhältnis von Allylalkohol zum Ester der Methacrylsäure im Bereich von 1:2,5 bis 1:5 liegt.

4. Verfahren gemäß mindestens einem der vorhergehenden Ansprüche, **dadurch gekennzeichnet, dass** 0,5 bis 2 mmol Zirkoniumacetylacetonat pro mol Allylalkohol eingesetzt werden.

5. Verfahren gemäß mindestens einem der vorhergehenden Ansprüche, **dadurch gekennzeichnet, dass** die Umsetzung bei einem Druck im Bereich von 500 bis 1.300 mbar erfolgt.

6. Verfahren gemäß mindestens einem der vorhergehenden Ansprüche, **dadurch gekennzeichnet, dass** die Umsetzung bei einer Temperatur im Bereich von 80°C bis 120°C erfolgt und dass die Temperatur, bei der die Umsetzung erfolgt, im Verlauf der Umsetzung erhöht wird.

7. Verfahren gemäß Anspruch 5 oder 6, **dadurch gekennzeichnet, dass** die Temperatur zu Beginn der Umsetzung im Bereich von 90°C bis 100°C und gegen Ende der Umsetzung im Bereich von 105 °C bis 115 °C liegt.

8. Verfahren gemäß mindestens einem der vorhergehenden Ansprüche, **dadurch gekennzeichnet, dass** die Umsetzung in Gegenwart eines Polymerisationsinhibitors, der keinen Stickstoff enthält, erfolgt.

9. Verfahren gemäß Anspruch 7 oder 8, **dadurch gekennzeichnet, dass** ein Phenol als Polymerisationsinhibitor eingesetzt wird.

10. Verfahren gemäß mindestens einem der Ansprüche 8 bis 9, **dadurch gekennzeichnet, dass** als Polymerisationsinhibitor eine Mischung verwendet, die Hydrochinon und Hydrochinonmonomethylether umfasst.

11. Verfahren gemäß mindestens einem der vorhergehenden Ansprüche, **dadurch gekennzeichnet, dass** der Wasseranteil im Allylmethacrylat höchstens 0,02% beträgt.

12. Verfahren gemäß mindestens einem der vorhergehenden Ansprüche, **dadurch gekennzeichnet, dass** die Reaktion unter Rühren stattfindet.

13. Verfahren gemäß mindestens einem der vorhergehenden Ansprüche, **dadurch gekennzeichnet, dass** die Umsetzung unter Einleitung von Luftsauerstoff erfolgt.

14. Verfahren gemäß Anspruch 13, **dadurch gekennzeichnet, dass** 0,05 bis 0,5 l Luft pro Stunde und Mol Allylalkohol eingeleitet wird.

15. Verfahren gemäß mindestens einem der vorhergehenden Ansprüche, **dadurch gekennzeichnet, dass** der aus dem eingesetzten Ester der Methacrylsäure freigesetzte Alkohol durch Destillation abgetrennt wird.

16. Verfahren gemäß Anspruch 15, **dadurch gekennzeichnet, dass** Ethanol oder Methanol abgetrennt wird.

17. Verfahren gemäß Anspruch 15 oder 16, **dadurch gekennzeichnet, dass** eine Mischung abgetrennt wird, die Methylmethacrylat und Methanol enthält.

18. Verfahren gemäß mindestens einem der Ansprüche 15 bis 17, **dadurch gekennzeichnet, dass** ein Teil der abgetrennten Mischung in den folgenden Ansatz eingeleitet wird.

19. Verfahren gemäß Anspruch 18, **dadurch gekennzeichnet, dass** der zurückführbare Anteil der abgetrennten Mischung gegen Ende der Reaktion gewonnen wird.

## Claims

1. Process for preparing allyl methacrylate, comprising the reaction of allyl alcohol with an ester of methacrylic acid, **characterized in that** the reaction is catalysed by zirconium acetylacetonate.

2. Process according to Claim 1, **characterized in that** the allyl methacrylate contains less than 0.02% allyl alcohol.

3. Process according to at least one of the preceding claims, **characterized in that** the weight ratio of allyl alcohol to the ester of methacrylic acid is in the range of 1:2.5 to 1:5.

4. Process according to at least one of the preceding claims, **characterized in that** 0.5 to 2 mmol of zirconium acetylacetonate are used per mole of allyl alcohol.

5. Process according to at least one of the preceding claims, **characterized in that** the reaction is effected at a pressure in the range of 500 to 1300 mbar.

6. Process according to at least one of the preceding claims, **characterized in that** the reaction is effected at a temperature in the range of 80°C to 120°C and **in that** the temperature at which the reaction is effected is increased in the course of the reaction.

7. Process according to Claim 5 or 6, **characterized in that** the temperature at the start of the reaction is in the range of 90°C to 100°C and is in the range of 105°C to 115°C towards the end of the reaction.

8. Process according to at least one of the preceding claims, **characterized in that** the reaction is effected in the presence of a polymerization inhibitor which does not contain nitrogen.

9. Process according to Claim 7 or 8, **characterized in that** a phenol is used as the polymerization inhibitor.

10. Process according to at least one of Claims 8 to 9, **characterized in that** the polymerization inhibitor used is a mixture which comprises hydroquinone and hydroquinone monomethyl ether.

11. Process according to at least one of the preceding claims, **characterized in that** the water content in the allyl methacrylate is at most 0.02%.

12. Process according to at least one of the preceding claims, **characterized in that** the reaction takes place with stirring.

13. Process according to at least one of the preceding claims, **characterized in that** the reaction is effected with introduction of atmospheric oxygen.

14. Process according to Claim 13, **characterized in that** 0.05 to 0.5 l of air per hour and mole of allyl alcohol is introduced.

15. Process according to at least one of the preceding claims, **characterized in that** the alcohol released from the ester of methacrylic acid used is removed by distillation.

16. Process according to Claim 15, **characterized in that** ethanol or methanol is removed.

17. Process according to Claim 15 or 16, **characterized in that** a mixture which comprises methyl methacrylate and methanol is removed.

18. Process according to at least one of Claims 15 to 17, **characterized in that** a portion of the mixture removed is introduced into the next batch.

19. Process according to Claim 18, **characterized in that** the recyclable portion of the mixture removed is obtained towards the end of the reaction.

## Revendications

1. Procédé de fabrication de méthacrylate d'allyle, comprenant la mise en réaction d'alcool allylique avec un ester de l'acide méthacrylique, **caractérisé en ce que** la réaction est catalysée par de l'acétylacétonate de zirconium.

2. Procédé selon la revendication 1, **caractérisé en ce que** le méthacrylate d'allyle contient moins de 0,02 % d'alcool allylique.

3. Procédé selon au moins l'une quelconque des revendications précédentes, **caractérisé en ce que** le rapport en poids entre l'alcool allylique et l'ester de l'acide méthacrylique se situe dans la plage allant de 1:2,5 à 1:5.

4. Procédé selon au moins l'une quelconque des revendications précédentes, **caractérisé en ce que** 0,5 à 2 mmoles d'acétylacétonate de zirconium sont utilisées par mole d'alcool allylique.

5. Procédé selon au moins l'une quelconque des revendications précédentes, **caractérisé en ce que** la réaction a lieu à une pression dans la plage allant de 500 à 1 300 mbar.

6. Procédé selon au moins l'une quelconque des revendications précédentes, **caractérisé en ce que** la réaction a lieu à une température dans la plage allant de 80 °C à 120 °C, et **en ce que** la température à laquelle la réaction a lieu est augmentée au cours de la réaction.

7. Procédé selon la revendication 5 ou 6, **caractérisé en ce que** la température se situe au début de la réaction dans la plage allant de 90 °C à 100 °C, et à la fin de la réaction dans la plage allant de 105 °C à 115 °C.

8. Procédé selon au moins l'une quelconque des revendications précédentes, **caractérisé en ce que** la réaction a lieu en présence d'un inhibiteur de polymérisation qui ne contient pas d'azote.

9. Procédé selon la revendication 7 ou 8, **caractérisé en ce qu'**un phénol est utilisé en tant qu'inhibiteur de polymérisation.

10. Procédé selon au moins l'une quelconque des revendications 8 à 9, **caractérisé en ce qu'**un mélange qui comprend de l'hydroquinone et de l'éther monométhylique d'hydroquinone est utilisé en tant qu'inhibiteur de polymérisation.

11. Procédé selon au moins l'une quelconque des revendications précédentes, **caractérisé en ce que** la proportion d'eau dans le méthacrylate d'allyle est d'au plus 0,02 %.

12. Procédé selon au moins l'une quelconque des revendications précédentes, **caractérisé en ce que** la réaction a lieu sous agitation.

13. Procédé selon au moins l'une quelconque des revendications précédentes, **caractérisé en ce que** la réaction a lieu sous introduction d'oxygène de l'air.

14. Procédé selon la revendication 13, **caractérisé en ce que** 0,05 à 0,5 l d'air par heure et mole d'alcool allylique est introduit.

15. Procédé selon au moins l'une quelconque des revendications précédentes, **caractérisé en ce que** l'alcool libéré à partir de l'ester de l'acide méthacrylique utilisé est séparé par distillation.

16. Procédé selon la revendication 15, **caractérisé en ce que** de l'éthanol ou du méthanol est séparé.

17. Procédé selon la revendication 15 ou 16, **caractérisé en ce qu'**un mélange contenant du méthacrylate de méthyle et du méthanol est séparé.

18. Procédé selon au moins l'une quelconque des revendications 15 à 17, **caractérisé en ce qu'**une partie du mélange séparé est introduit dans la préparation suivante.

19. Procédé selon la revendication 18, **caractérisé en ce que** la proportion recyclable du mélange séparé est obtenue à la fin de la réaction.
